# EUROPEAN PATENT SPECIFICATION

(11) **EP 1 377 699 B1**
(45) Date of publication and mention of the grant of the patent: **19.09.2007**
(21) Application number: 02724191.8
(22) Date of filing: 04.03.2002
(51) Int. Cl.: D01F 4/00, A61L 15/32, A61L 27/00

(54) **ELONGATED BIOPOLYMER STRUCTURE CONTAINING FIBRIN**
FIBRIN ENTHALTENDE LANGGESTRECKTE BIOPOLYMERSTRUKTUR
STRUCTURE BIOPOLYMERE ALLONGEE RENFERMANT DE LA FIBRINE

(30) Priority: 06.03.2001 US 800070
(43) Date of publication of application: 07.01.2004
(73) Proprietor: Baxter Healthcare S.A., 8304 Wallisellen (CH); Baxter International Inc., Deerfield, Illinois 60015 (US)
(72) Inventor: DELMOTTE, Yves, Alain, B-732 Neufmaison (BE)
(74) Representative: Bassett, Richard Simon
(86) International application number: PCT/EP2002/002315
(87) International publication number: WO 2002/070795

(56) References cited:
- EP-A- 0 344 924
- EP-A- 0 485 210
- EP-A- 0 998 949
- US-A- 2 576 006
- US-A- 4 442 655

## Description

### FIELD OF THE INVENTION

The invention pertains to articles useful for directing wound healing and cell growth, particularly in the field of tissue engineering.
The articles are made from a stretched structure containing fibrin.

### BACKGROUND OF THE INVENTION

Fibrin and fibrinogen material have been used for making various products since a long time. For example, fibrin sponges are known from US 4,442,655 and WO 99/15209. Said fibrin sponges have a porous structure and have substantially no compression strength. After hydration, the sponges have pores with a cross-section area of less than 100 µm², i.e. a porosity which is different from the porosity of a human natural bone.

From the WO 99/27167 a process for making fibronectin or fibrinogen fibers and articles comprising those fibers is known. This process includes an extrusion of a protein solution through an orifice.

From the WO 92/13003 a porous macroscopically oriented cell adhesion protein like e.g. fibronectin, vitronectin or von Willebrand protein is known. Again this orientation is achieved in that the forming and orienting of said cell adhesion protein fibrils is carried out from a solution and the solvent is then removed.

EP 0 485 210 discloses a fibrin/collagen membrane material for biomedical use which may be dried in a stretched configuration.

US 2,576,006 discloses a fibrous film that may be made into shaped products in which the film can be stretched to conform to the new shape.

### SUMMARY OF THE INVENTION

The present invention provides new materials for directing wound healing processes and cell growth. These materials are especially useful in the field of tissue engineering. The materials of the invention are made from an elongated biopolymer structure containing fibrin, the structure having at least one stretched portion in only one stretching direction. The elongated biopolymer structure can also contain additional materials such as fibrinogen, polysaccharides, or collagen. The materials made from this structure are formed into threads or tubes. Other hollow profiles, films, fleeces, sponges and membranes are also provided, but do not form part of the invention.

### BRIEF DESCRIPTION OF THE DRAWINGS

Figure 1 is a cross-section view of a fibrin-containing thread (not stretched).
Figure 2 is an enlarged view of the outer wall of the thread of Figure 1.
Figure 3 is a cross-section view of the thread of Figure 1.
Figure 4 is a cross-section view of the thread of Figure 1, after stretching in air.
Figure 5 is an enlarged view of the outer surface of the thread of Figure 4.
Figure 6 is an enlarged view of the cross-section.
Figure 7 is a cross-section view of the thread of Figure 1, after stretching in a fixative solution.
Figure 8 is an enlarged view of the outer surface of the thread of Figure 7.
Figure 9 is an enlarged view of the cross-section of the thread of Figure 7.

Figure 10 is a perspective view of a fibrin containing tube (not stretched).

Figure 11 is a cross-section view of the tube of Figure 10.

Figure 12 is an enlarged view of the inner wall of the tube of Figure 10.

Figure 13 is an enlarged view of the outer wall of the tube of Figure 10.

Figure 14 is an enlarged cross-section view of the tube of Figure 10 at the injection point.

### BRIEF DESCRIPTION OF THE INVENTION

The invention is an elongated biopolymer structure containing fibrin, as defined in claim 1, which structure has at least one portion stretched in only one stretching direction. The structure is useful in the construction of articles for directing wound healing and cell growth, particularly in the field of tissue engineering. In addition to fibrin, the elongated biopolymer structure can also contain a second material such as fibrinogen, chondroitin-4 sulfate, dermatan sulfate, keratan sulfate, alginate, laminin, elastin, fibronectin, collagen, organic polymer, peptide, and N-containing polysaccharides such as hyaluronic acid, N-acetyl-glucosamines, chitins, and chitosans, especially N,O-carboxymethylchitosan, derivatives thereof and mixtures thereof.

Herein, the term "elongated biopolymer structure" refers to a structure made from fibrin with or without other materials added.

Preferably; the stretched portion of the structure is porous. Advantageously, the elongated structure is a stretched portion of a porous fibrin.

The stretched portion preferably has at least two densities which are different from each other. Preferably the first density is at least 1.5 times, advantageously at least 5 times the second density. In another preferred embodiment the first density is at least 2 times the first density.
The stretched portion can for example have a first surface density, which is substantially parallel to the stretching direction in a surface section parallel to the stretching direction and by a second surface density substantially parallel to the stretching direction in a surface section perpendicular to the stretching direction, said first surface density being higher than said second surface density.
The elongated structure or the article comprising such a structure has a shape selected from the group consisting of thread or tube. Other hollow profile, film, fleece, sponge and membrane is also provided. Herein, the term "hollow profile" refers to a form having a hollow space, such as a tube; the term also includes rectangular, hexagonal, pentagonal, octagonal or irregularly shaped forms which are useful for applications in various specific areas of a patient's body, for instance.
The invention relates to mono-layer products as well as to multi-layer products. In a preferred embodiment, the multi-layer product has at least one layer which consists at least partly of stretched elongated fibrin structure.
The invention also relates to a process for the preparation of an elongated biopolymer structure having at least a portion stretched in only one stretching direction. This process comprises the following steps:
(a) providing a first component of a fibrinogen-containing material;
(b) providing a second component of a substance having a capability to convert fibrinogen into fibrin;
(c) forming a fibrin-containing material by mixing the first component with the second component; and
(d) subjecting the fibrin-containing material to stretching in only one direction to obtain the elongated biopolymer structure.

In another embodiment of the process, a second biopolymer is added either between steps (a) and (b) or between steps (b) and (c). The second biopolymer may be already stretched when added, or it may be in unstretched form when added.

In a preferred embodiment the stretching is sufficient to elongate the length of the structure by at least 5%, advantageously by at least 20%, preferably by at least 50%, most preferably by at least 100%.

According to another process of the invention the substance having a capability to convert fibrinogen into fibrin is thrombin, preferably a thrombin in an inactive form. Such an inactive thrombin can be a photoactivatable thrombin, which is put into in the active form by treatment with energy, e.g. light. An example for such a thrombin is disclosed in US 5,318,524, the content of which is incorporated herein by reference.

A process using such thrombin in an inactive form for forming the fibrin can comprise the following steps: An aqueous solution containing fibrinogen and thrombin in an inactive form is provided. The amount of water present in the solution is adapted, so that after activation of the inactive thrombin, a polymerized gel is formed, said gel having a water content such that there is substantially no water removal when submitting the gel to a centrifugation of 2500 rounds per minute. Said polymerized gel is then submitted to a stretching step, said stretching being sufficient to elongate the length of the preferably shaped product of at least 5%, advantageously at least 20%, preferably at least 50%, most preferably at least 100%.

A further object of the invention is to provide articles e.g. fabrics, especially knitted fabrics, which comprise or are made at least partly of an elongated biopolymer structure having at least a portion stretched in only one stretching direction. The shapes are selected from a thread or a tube. Other hollow profile, film, fleece, sponge or membrane is also provided. The stretched portion preferably has a diameter of less than 10 mm, more preferred less than 5 mm, advantageously of less than 3 mm, most preferably between 250 µm and 2500 µm.

### DETAILED DESCRIPTION OF THE INVENTION

The invention is an elongated biopolymer structure containing fibrin, which structure has at least a portion stretched in at only one stretching direction.
In addition to fibrin, the structure of the invention can also contain material selected from the group consisting of fibrinogen, chondroitin-4 sulfate, dermatan sulfate, keratan sulfate, alginate, laminin, elastin, fibronectin, collagen, organic polymer, peptide, and N-containing polysaccharides such as hyaluronic acid, N-acetyl-glucosamines, chitins, and chitosans, especially N,O-carboxymethylchitosan, derivatives thereof and mixtures thereof.
The fibrin and the additive material can be of natural origin (e.g. animal or human source material), a recombinant product, a genetically produced or modified product, a synthetically made one or one from a mixture of these sources. Preferably the fibrin material is human in nature, that is the fibrin precursor components, fibrinogen and thrombin, are either human-plasma-derived or produced by genetic engineering of cells or animals to yield fibrinogen and thrombin that are either identical to or similar to the respective human proteins.
Preferably, the stretched portion of the structure is porous. The elongated structure of the invention advantageously has a porous stretched portion, that is characterized by an orientation of the bio-polymer, said orientation being such that there are at least two densities which are different from each other. Differences in densities may be determined by common known methods like e.g. examination by scanning electron microscopy=SEM. A first density and a second fibrin density can then be determined, said first fibrin density being higher than said second fibrin density. Preferably, the first density (e.g. as determined by SEM view) is at least 1.5 times the second fibrin density, preferably at least 2 times the second density, most preferably at least 5 time the second density. For a structure stretched in one direction, it means that for a surface or slice or cross-section view parallel to a stretching direction, the fiber density is higher than the fiber density in a slice or cross-section view perpendicular to the stretching direction.
The stretched portion can have different shapes selected from the group consisting of thread or tube. Other hollow profile, film, fleece, sponge, membrane is also provided, or a combination thereof. The stretched portion is for example a layer (mono-layer product) or a multi-layer product or a part thereof. Preferably, the stretched portion has a shape selected from the group consisting of thread or tube or a layer thereof, said stretched portion having an outer equivalent diameter of less than 10 mm, advantageously of less than 5 mm, more advantageously of less than 3 mm and is preferably comprised between 150 µm and 2500 µm. In case of a circular cross-section, the equivalent diameter is equal to the outer diameter of the thread or tube. In case the thread, tube or hollow profile has a non-circular cross-section, the outer equivalent diameter is equal to (4 S/P) in which P is the length of the outer perimeter of the cross-section perpendicular to the longitudinal axis of the thread or tube, while S is the surface defined within said outer perimeter.
In the case of hollow profile with a central channel substantially parallel to the stretching direction, the central channel has a cross-section perpendicular to the stretched direction with an equivalent diameter of less than 15 mm, advantageously of less than 10 mm, preferably of less than 5 mm, most preferably comprised between 150 µm and 2500 µm. In case of a circular cross-section for the hollow profile or channel, the equivalent diameter is equal to the diameter of the channel. In case the channel has a non-circular cross-section, the equivalent diameter is equal to (4 S/P) in which P is the length of the cross-section perimeter of the channel (cross-section perpendicular to the stretching direction), while S is the surface defined within said perimeter. The hollow profile or the tube has, for example, a wall thickness comprised between 0.1 mm and 5 mm, advantageously between 0.25 mm and 2.5 mm, preferably between 0.5 and 2 mm.

In an embodiment, the stretched portion is at least provided with an element selected from the group consisting of various shapes prepared from non-biopolymer containing material. Such non-biopolymer containing material are for example fibers such optical fibers, electrical conductive fibers, etc.

The structure can be made from an at least partly cross-linked material. According to an advantageous embodiment, the stretched portion has an outer surface consisting of biopolymer, preferably of fibrin, at least partly cross-linked by means of a cross-linking agent, such as factor XIII, tannic acid, aldehydes, advantageously a formaldehyde, a glutaraldehyde, or a mixture thereof.

According to another possible embodiment, the stretched portion forms the coating of an elongated element, such as for example a catheter.

According to a preferred embodiment, the biopolymer is a fibrin or fibrinogen based biopolymer, possibly containing one or more biopolymer of at least a compound selected from the group consisting of chondroitin-4 sulfate, dermatan sulfate, keratan sulfate, hyaluronic acid, chitosan, chitin, alginate, laminin, elastin, fibronectin, collagen, organic polymer, peptide, derivatives thereof, and mixtures thereof.

The elongated biopolymer structure of the invention can also contain or be mixed (for example after or during a re-hydration step) with one or more additives, such as a compound selected from the group consisting of protein, genetic material, anticoagulant, inorganic compound, growth factor, cells, anti-inflammatory compound, compound reducing graft rejection, cell growth inhibitor, antibiotic, antiseptic, analgesic, antineoplastic, chemotherapeutic, polypeptide, protease inhibitor, vitamin, cytokine, cytotoxin, interferon, hormone, antibody, antimicrobial agent, agent for improving the biocompatibility, and mixtures thereof.

Cells which are added can be, for example, osteoblasts, osteoclasts, chondroblasts, chondrocytes, keratinocytes, fibroblasts, hematopoeitic cells, stromal cells, neural cells, diverse tissue progenitor cells or stem cells.

The biopolymer structure can also contain processing aids such as compounds which help the stretching operation. Examples of processing aids are compounds having lubricating properties, oils, fatty acid, etc.

The invention relates also to a process for the preparation of a fibrin comprising an elongated structure of a biopolymer.
This process comprises the following steps:
(a) providing a first component of a fibrinogen-containing material;
(b) providing a second component of a substance having a capability to convert fibrinogen into fibrin;
(c) forming a fibrin-containing material by mixing the first component with the second component; and
(d) subjecting the fibrin-containing material to stretching in only one direction to obtain the elongated biopolymer structure.

In another embodiment of the process, a second biopolymer is added either between steps (a) and (b) or between steps (b) and (c). The second biopolymer may be already stretched when added, or it may be in unstretched form when added.

The stretching preferably is sufficient to elongate the length of the product of at least 5%, advantageously at least 10%, preferably at least 25%, most preferably at least 50%, although 100% is also preferred.

Preferably a mechanical or a physical treatment is carried out to achieve an elongation of the material. Examples of a mechanical treatment include compression or extrusion; examples for a general physical treatment include energy treatment, like radiation with e.g. light, or processes like freeze-drying.

Advantageously, after the stretching and/or during the stretching, the fibrin-comprising product is submitted to a drying step. The drying can be operated in air (preferably sterile air) or an oxygen-containing atmosphere (preferably sterile), but is preferably carried out in an inert atmosphere, such as nitrogen atmosphere.

Advantageously, the fibrin-comprising material is prepared under pressure, so as to limit the formation of void volume.

Advantageously, the fibrin-comprising material is prepared in a mould or in dies in which the material is thereafter subjected to the above-mentioned mechanical or physical treatment(s). For example, the starting material used for the preparation of the structure of the invention is at least partly polymerized and/or cross-linked in a mould or dies in which the material thereafter is subjected to an appropriate mechanical or physical treatment.

According to a preferred embodiment, the fibrin-comprising product, preferably compressed and shaped, is at least partly dried during the stretching procedure. Advantageously the drying procedure is finished after the stretching operation.

After the drying procedure the structure and/or final product of the invention, i.e. the elongated biopolymer structure, has advantageously a low moisture content, for example a moisture content of less than 7.5%, preferably of less than 2%, most preferably of less than 1%, and more specifically less than 0.5%.

According to another embodiment, the fibrin-comprising material is submitted to a water removal before the stretching procedure is carried out. For example, the minimal water removal is such that when submitting the clot to a centrifugation at 1000 rounds per minute substantially no water is further removed. Advantageously, the minimal water removal is such that when submitting the material to a centrifugation at 2,000 rounds per minute, substantially no water is further removed. Preferably, substantially all the free water of the fibrin-comprising material is removed. The removal can be carried out by a heating step, by an air-drying step, but is preferably carried out by filtration, by osmosis or by centrifugation. A preferred method is a centrifugation combined with a filtration. For example, the clot is submitted to a centrifugation - filtration at a speed of more than 1000 rounds per minute, advantageously of more than 2000 rounds per minute, preferably of more than 2500 rounds per minute.

According to an advantageous embodiment, the fibrin-comprising product is at least partly stretched in a solution containing a cross-linking agent, which causes a cross-linking of the biopolymer during the stretching. The cross-linking agent is for example an aldehyde, such as a formaldehyde, a glutaraldehyde, or a mixture thereof.

Although the cross-linking can be conducted after the stretching procedure, it is advantageous to conduct the cross-linking during the stretching, so as to reinforce the links between the biopolymer/s during the stretching.
Advantageously, the fibrin-containing material is cast and compressed in dies or in a mould so as to obtain a compressed fibrin-containing product having a shape selected from the group consisting of thread or tube. Other hollow profile, film, fleece, sponge and membrane shapes are also provided.
When using moulds or dies for the substantially continuous extrusion of the fibrin-containing material, the moulds or dies will advantageously have a length permitting a sufficient polymerization of the clot.
In a preferred embodiment the mould or dies are transparent, so that energy like light can pass through.
According to one embodiment, the fibrin-comprising material contains at least a further polymerizable or cross-linkable compound, such as one or more compounds selected from the group consisting of fibrinogen, chondroitin-4 sulfate, dermatan sulfate, keratan sulfate, hyaluronic acid, chitosan, chitin, alginate, laminin, elastin, fibronectin, collagen, organic polymer, peptide, derivatives thereof, and mixtures thereof.
To form the fibrin or fibrinogen-containing material, advantageously a solution containing thrombin, advantageously less than 1000 IU/ml thrombin, preferably less than 200 IU/ml thrombin, most preferably less than 100 IU/ml thrombin together with a fibrin-fibrinogen solution are used. When thrombin in an inactive form is used (i.e. thrombin which can be activated for example by a radiation), a larger amount of thrombin can be used than with the thrombin in active form. When using active thrombin, the structure is advantageously prepared from a fibrinogen solution containing less than 50 IU/ml thrombin, preferably less than 10 IU/ml thrombin, most preferably less than 1 IU/ml thrombin.

The thrombin can be of natural origin, i.e. derived from human or animal plasma, recombinant thrombin, a synthetic peptide, a thrombin in inactive form, which can be activated e.g. by radiation or light (=photoactivatable thrombin), a derivative thereof or a mixture thereof.

When using thrombin in an inactive form, such as a photoactivatable thrombin (i.e. an inactive thrombin which can be activated by light of specific wavelengths), it is possible to activate the thrombin while submitting the fibrinogen solution to a treatment effecting a compacting of the structure, e.g. compression via a centrifugation procedure.

Advantageously, the fibrin-comprising material is prepared from a fibrinogen-containing solution containing at least 3 mg/ml fibrinogen, advantageously at least 5 mg/ml, preferably at least 10 mg/ml fibrinogen material, most preferably at least 20 mg/ml, for example from 50 mg/ml up to 250 mg/ml or from 50 up to 150 mg/ml.

According to an embodiment, the fibrin-comprising material or structure is prepared from a fibrinogen-containing solution containing a calcium complexing (or chelating) agent. The presence of a sufficient amount of calcium complexing agent for inhibiting or blocking substantially all the calcium sites enables the preparation of a biopolymer-containing material provided with a low cross-linking rate. Advantageously an additional cross-linking can be carried out in presence of at least a cross-linking agent.

The cross-linking agent can percolate through the product during compression, for example during its centrifugation.

According to one embodiment, the fibrin-comprising material is prepared from a solution containing fibrinogen plus possibly preformed fibrin, and an additional material selected from chondroitin-4 sulfate, dermatan sulfate, keratan sulfate, hyaluronic acid, (in general polysaccharides) chitosan, chitin, alginate, laminin, elastin, fibronectin, collagen, organic polymer, peptide, derivatives thereof, and mixtures thereof, plus at least one additive selected from the group consisting of protein, genetic material, anticoagulant, inorganic compound, growth factor, cells, anti-inflammatory compound, compound reducing graft rejection, cell growth inhibitor, antibiotic, antiseptic, analgesic, antineoplastic, chemotherapeutic, polypeptide, protease inhibitor, vitamin, cytokine, cytotoxin, interferon, hormone, antibody, antimicrobial agent, agent for improving the biocompatibility, and mixtures thereof.

According to another embodiment, after preparing the fibrin containing solution, before a treatment like e.g. compression of the biopolymer-comprising material is carried out, at least an additive is added to this product.

According to one embodiment, the stretched product is submitted to a lyophilization step so as to prepare a dry or substantially dry porous support.

According to another process of the invention, a thrombin in inactive form (such as a photoactivatable thrombin) is used. In said process, an aqueous solution containing fibrinogen-containing material and thrombin in an inactive form is provided. The amount of water present in the solution is adapted, so that after activation of the inactive thrombin, a polymerized gel is formed, said gel having a water content such that there is substantially no water removal when submitting the gel to a centrifugation of 1000 rounds per minute (advantageously 2,000 rounds per minute, preferably more than 2,500 rounds per minute). Said polymerized gel is then submitted to a stretching, said stretching being sufficient to elongate the length of the shaped product of at least 5%, advantageously at least 20%, preferably at least 50%, most preferably at least 100%.

Advantageously, during the polymerization step, the aqueous solution is submitted to compression. Such compression is advantageous for preventing the formation of voids in the polymerized product.

The activatable thrombin and the fibrinogen are for example first mixed in a dry form. Then, water is added to the dry mixture of particles of activatable thrombin and fibrinogen. The so formed mixture can then be cast or extruded. When making an extrusion through dies, the activatable thrombin is advantageously activated when the mixture passes through the dies or just before the mixture passes through the dies. The amount of water added or used is advantageously adapted, so that after polymerization, no water can be removed when centrifugating the polymerized material at a speed of 1000 rounds per minute, preferably of more than 2000 rounds per minutes, most preferably of more than 2,500 rounds per minutes.

Additives can be added to the aqueous solution containing the activatable thrombin and the fibrinogen material. Said additives are for example mixed in a dry form with the dry activatable thrombin and/or the dry fibrinogen material, or can be added to water before adding said water to the thrombin and/or to the fibrinogen and/or to the mixture thrombin/fibrinogen.

The aqueous solution containing thrombin and fibrinogen can contain up to 1000 IU/ml activatable thrombin.

Possible additives for the solution are for example further polymerizable or cross-linkable compounds, such as one or more compounds selected from the group consisting of fibrin, fibrinogen, chondroitin-4 sulfate, dermatan sulfate, keratan sulfate, hyaluronic acid, chitosan, chitin, alginate, laminin, elastin, fibronectin, collagen, polysaccharides, organic polymers, peptides, derivatives thereof, and mixtures thereof.

Additives which can be added before and/or after the polymerization and/or after a stretching step are for example: antibody, antimicrobial agent, agent for improving the biocompatibility of the structure, proteins, anticoagulants, anti-inflammatory compounds, compounds reducing graft rejection, living cells, cell growth inhibitors, agents stimulating endothelial cells, antibiotics, antiseptics, analgesics, antineoplastics, chemotherapeutics, polypeptides, protease inhibitors, vitamins, cytokine, cytotoxins, minerals, proteins, interferons, hormones, polysaccharides, genetic materials, proteins promoting or stimulating the growth and/or attachment of endothelial cells on the cross-linked fibrin, growth factors, cell growth factors, growth factors for heparin bond, tannic acid, nerve growth factor, neurotrophic factor (NTFs), neurothrophin 3 (NT3), brain derived NTF (BDNTF), cilary NTF (CNTF), substances against cholesterol, pain killers, collagen, osteoblasts, chondroblasts, chondrocytes, osteoclasts, hematpoeitic cells, stromal cells, osteoprogenitor cells, drugs, anti coagulants, poly DL lactate, alginate, recombinant material, triglycerides, fatty acids, C₁₂-C₂₄ fatty acids, substances against cholesterol, pain killers, collagen, drugs, activatable (preferably light activatable) factor VII, activatable (preferably light activatable) factor IX, activatable (preferably light activatable) factor X, activatable (preferably light activatable) factor XI, activatable plasmin, photoactivatable t-PA, photoactivatable urokinase, and mixtures thereof.

After the polymerization step, for example after the passage of the fibrinogen-thrombin solution through the extrusion dies, the shaped product is advantageously dried and/or submitted to a lyophilization step.

The fibrinogen-containing solution has for example a fibrinogen content of at least 5 mg/ml, advantageously at least 10 mg/ml, preferably at least 20 mg/ml, most preferably at least 50 mg/ml fibrinogen material (for example from 50 up to 250 mg/ml fibrinogen material).

According to another process, particles of activatable thrombin and particles of fibrinogen material are dry-mixed together and are compacted. Thereafter water is added to the compacted dry mixture. Then the wet compacted particles are submitted to a treatment for activating the activatable thrombin, so as to start the polymerization. Possibly dry or substantially dry particles containing fibrinogen material and thrombin are first prepared, said particles being thereafter compacted (for example in a mould or dies). The compacted particles are then wetted. Thereafter, the wetted compacted particles are submitted to a treatment for activating the thrombin, so that the polymerization of the fibrinogen material can be started.

When using photoactivatable thrombin, the polymerization can be controlled by the intensity of lights applied on the thrombin, light having a specific wavelength or having a specific range of wavelengths. The polymerization will also depend from the polymerization time and the temperature of the polymerization. By controlling the light intensity or the energy of the light source applied, it is possible to control the polymerization of the fibrinogen, and/or the cross-linking. By controlling the light intensity, it is then possible to control the thickness and/or the porosity of the layer.

Multi-layered material can be prepared by using this technique. For example, a three layered material having external layers characterized by tight pore size and an intermediate layer characterized by an open structure. Multi-layered product can therefore be manufactured for preventing adhesion or for promoting wound healing.

Due to the possibility to define the area or zone where a polymerization has to be initiated by irradiating the photoactivatable thrombin (for activating the thrombin of the illuminated area or zone) it is possible to manufacture fibrin elements or pieces by using a three dimensional printing process, for example by using a MIT Alpha Machine. Three-dimensional printing functions by building parts in layers. When applied to the thrombin-fibrinogen particles, a thin layer of particles is spread over the surface of a powder bed. The layer is wetted and by means of a computer, area of the layer is illuminated at the place where a part has to be built. At the illuminated area fibrinogen material is polymerized, advantageously with an at least partial cross-linking. Thereafter a further layer of thrombin-fibrinogen material is spread on the previous layer, and, after wetting, the layer is illuminated at the place where fibrinogen material has to be polymerized. Possibly, instead of using dry powders, it is possible to use a solution of inactivated thrombin (activatable) and fibrinogen material.

In order to avoid possible undesired polymerization, the superimposed layers are kept at low temperature, for example at a temperature at which substantially no polymerization is possible. Therefore, the light radiation of an area or zone of a layer will be sufficient for activating the thrombin of said area or zone but also for elevating the temperature of said area or zone for enabling the polymerization. Possibly, two or more than two different radiation are used, a first for activating the thrombin, another for increasing the temperature. By controlling the intensity or energy of light applied to the layer or to portions of the layer, it is possible to control the structure and/or the porosity of the layer. The so prepared product comprises thus a series of superimposed fibrin containing layer, each layer having a structure and porosity which can vary in the layer, and which can be the same or different from the structure and porosity of one or more adjacent fibrin containing layers.

According to another possible process, a solution of fibrinogen and inactive thrombin is prepared. The thrombin is activated in the solution by a means enabling a local activation of the thrombin so as to form locally fibrin bonds. The activation (energy, density of activation, etc.) can be controlled so as to control the structure or porosity of the local polymerized fibrin element. By successive local activation, it is possible to form successive fibrin elements. When the successive local activation is carried out so that two activations, preferably successive activations, are adjacent, fibrin elements can be connected to each other so as to form the product. The various elements forming the product can have the same or different structure and/or porosity and/or thickness.

Advantageously, the thrombin is a thrombin, which can be activated by light. In this case, the fibrinogen-inactive thrombin solution is placed in a chamber or area in which the solution is protected from undesired light. By using a light source, for example a focalized light source or a laser, emitting light having the necessary characteristic for activating the thrombin (for example having a specific wavelength or having a wavelength within a specific range and/or having a specific energy density and/or a specific band width), the polymerization of fibrinogen in the solution can be initiated area by area, preferably layer by layer. Depending on the energy of the light knife therefore the layer will have a defined thickness and porosity.

So multi-layered material can be obtained, said layer having different structure (open/dosed) and/or different porosity and/or different properties and/or different pore size or density.

According to a possible embodiment, the fibrinogen-inactivate thrombin solution is moved or displaced with respect to a light source or a means for activating the thrombin. In this embodiment, the structure of the fibrin can be modified by controlling the characteristic of the source (energy, wavelength, density, etc.) and/or by controlling the movement of the solution (for example of the flow) with respect to the source.
According to another embodiment the thrombin is an inactivated thrombin requiring at least two lights of different wavelengths for being activated. For example, the activation of the thrombin requires a first light with a first wavelength or band of wavelength and a second light with a second wavelength or band of wavelength. With such a thrombin, the activation in a solution can be easily be made locally, namely at the intersection of the first light with the second light. When using lasers, the polymerization can be carried out substantially points by points in the solution.
The invention relates also to articles or fabrics, especially knitted fabrics comprising elongated structures with stretched portion selected from the group consisting of stretched thread or tube, said stretched portion having a diameter of less than 10 mm, advantageously of less than 5 mm, more preferred less than 3 mm and preferably comprised between 250 µm, and 2500 µm, and/or made at least partly from stretched element(s) selected from the group consisting of the before listed shapes. The article or fabrics can possibly be made of non-stretched elements and be stretched thereafter.
The articles or fabrics are advantageously provided with at least an additive as already mentioned earlier.
Advantageously, the fibrin-comprising elements are cross-linked after the preparation of the articles or fabrics. This is advantageous for creating links between adjacent elements, for example at the crossing points thereof.
We also provide a fibrin-containing film, fleece, sponge or membrane provided at least with one stretched portion in only one direction.
The film, fleece, sponge or membrane can be for example rolled around an axis perpendicular to a stretching direction. Possibly the material can be submitted to a cross-linking step after its stretching and/or during its stretching (preferably during its stretching).
The articles or fabrics of the invention and/or the thread or tube of the invention, or other hollow profile film, fleece, sponge or membrane provided herein can be associated with another biopolymer shape, e.g. a sponge, with one or more additives, for example in the form of a paste, etc or with a different biomaterial of any shape.
The stretched portion according to the invention, the articles or fabrics of the invention, and the various shapes according to the invention can be used for making cell supports, e.g. for a bioreactor, wound repair means, "artificial" skin, artificial vessel or any other type of tissue.
Preferably, the mechanical properties of the structure of the invention are at least partly kept after re-hydration.
Advantageously, the structure after further cross-linking with a cross-linking agent, has fibrin or fibrinogen bonds such that, after hydration, the ratio volume of hydrated structure/volume of the dry structure is comprised between 0.5 and 1.5, advantageously between 0.7 and 1.2, preferably between 0.9 and 1.1, most preferably about 1.
Preferably, the atomic ratio Ca/P of the structure is adjusted. For example, the said atomic ratio is comprised between 0.5 and 5, preferably lower than 2, most preferably comprised between 1.67 and 1.95. The adjustment of said ratio can be carried out by adding to the biopolymer (possibly after or during the stretching and/or before the stretching) or to the solution used for forming the biopolymer.

The structure of the invention, the articles or fabrics of the invention as well as all the materials of various shapes of the invention can be sterilized, by treatment with sterilizing agent, by Gamma radiation, by X-rays, with ethylene oxide, heating, etc. The treatment is preferably a treatment that does not denature the biopolymer, or at least does not denature the fibrin in the elongated biopolymer structure. When sterilizing with Gamma radiation, the sterilization is advantageously carried out at a temperature below 0°C, for example at a temperature below -25°C.

Although the sterilization step is preferably carried out after the stretching step, the fibrin - comprising, preferably already shaped product, can be sterilized before the mechanical or physical treatment, e.g. compression step and/or after this treatment but before the stretching step.

In another preferred embodiment the components for making the material according to the present invention is virus-safe, essentially free of contaminations and also screened according to state of the art procedures in the field of the present invention. Especially when the components are of natural origin, like e.g. out of blood or plasma, at least one virus inactivation or depletion procedure is carried out either before making the appropriate product or afterwards.

The structure of the invention, the articles and various products of the invention are advantageously pyrogen-free.

The structure of the invention can be provided with at least a binding agent, for example in the form of a layer, on at least a part of their surface.

The structure of the invention, the article or fabrics of the invention as well as the material of all the various shapes of the invention can comprise or can be associated with at least an additive as mentioned earlier.

The material of the invention can be manufactured from a fibrinogen-containing material further containing bone particles or bone chips. According to a preferred embodiment, bone chips, such as bone chips or particles having an average particle size (average by weight) lower than 2 mm, for example from 100 µm to 1 mm, advantageously from about 250 µm to 750 µm, preferably about 500 µm.

The structure of the invention can also be coated with bone chips, for example by means of a glue or cement. The glue or cement used can be any suitable and compatible glue or cement, said glue or cement possibly containing additive(s), active agent(s), etc.

Still a further product of the invention consists of a laminated product comprising at least a layer having the structure according to the invention, especially at least a layer made of an article (fabric) of the invention and/or at least one of the shapes according to the invention. For example, the product comprises several layers having a structure of the invention or a layer with the structure of the invention forming an intermediate layer extending between two layers. The said laminated product can possibly be compacted.

Still a further object of the invention is a multi-layer structure, said multi-layer structure comprising at least a structure of the invention. The multi-layer structure of the invention can for example comprise a first layer having a structure according to the invention, and a second layer selected from the group consisting of a structure according to the invention which is different from the first layer, a skin layer, a porous layer, a sponge layer, preferably the said second layer is also a fibrin or fibrinogen layer.

According to an embodiment in which a calcium inhibiting or blocking agent is present during the formation of the fibrin-containing material, the calcium inhibiting or blocking agent is selected from the group consisting of citrate salts, phosphate salts, oxalate salts and mixtures thereof. Other possible agents are compounds blocking calcium sites of the fibrinogen, such as sodium polyphosphate, zeolithe, phosphate, potassium citrate, salt of phosphonic acid, amine, glycol, diethyleneglycol, triethanolamine, ammonium, betaine, glycerophosphate, aluminosilicate (zeolithe P), hexametaphosphate, polyacrylate, oligomeric phosphates, polyethyleneglycol, tannic acid, verapamil salt, piperidine dion derivatives, guanidine derivatives, amlodipine benzenesulfonate, 3-methylflavone-8-carboxylic acid esters, compounds having antagonist properties towards calcium ion, lidoflazine, etc., anticoagulants such as dextran, 2,6 dimethyl 4(2nitrophenyl) 1,4 dihydro pyridine 3,5 dimethyl dicarboxylate (Nifedipine), heparin, ((diphenylacetyl-4,5 oxazolyl-2)immino)-2,2' diethanol, trombarin, bisobrine, morphine, amphetamine, antibiotics having anti coagulant properties, sodium salt of the 4 amino 2 hydroxybenzoic acid, sodium citrate and mixtures thereof.

Preferably, the calcium blocking agent is also an anticoagulant such as dextran, 2,6 dimethyl 4(2nitrophenyl) 1,4 dihydro pyridine 3,5 dimethyl dicarboxylate (Nifedipine), heparin, ((diphenylacetyl-4,5 oxazolyl-2)immino)-2,2' diethanol, trombarin, bisobrine, morphine, amphetamine, antibiotics having anticoagulant properties, sodium salt of the 4 amino 2 hydroxybenzoic acid, sodium citrate and mixtures thereof.

The partial or complete clotting of the fibrinogen-containing material (polymerization, with a partial or substantially complete cross-linking) is advantageously carried out at a pH between 6 and 10, more preferably between 7 and 8, most specifically at about 7.5. In order to ensure a substantially constant pH during the cross-linking, one or more buffers can be used. Preferably, when only a partial cross-linking in the material is intended, the buffer used has preferably a calcium inhibiting action.

Possibly, the partial or complete polymerization, advantageously with an at least partial cross-linked material is carried out at a pH lower than 6, but preferably at a pH sufficient for avoiding the denaturation of the fibrin or fibrinogen-containing material. For example, the polymerization is carried out at least partly at a pH of 4 - 6, preferably of 5 - 5.5. In this case, the calcium inhibiting or blocking agent is an acid, advantageously an organic acid, for example organic acid with 1 to 24 carbon atoms, most specifically citric acid, acetic acid, formic acid, tannic acid, etc., citric acid being preferred.

According to a possible embodiment, the polymerization or clotting is carried out in a first step at a first pH lower than 6 - 6.5, and in a second step at a second pH higher than the first pH. For example, in the second step, the polymerization is carried out at a pH comprised between 6 and 10, advantageously between 6.5 and 8, preferably at a pH comprised between 7 and 7.5, most specifically at a pH of 7.1-7.4.

The polymerization, advantageously with partial cross-linking, or clotting is carried for example at a temperature from about 0°C to 60°C, advantageously at a temperature from 5°C to 50°C, preferably at a temperature from 20°C to 40°C, most preferably at a temperature of about 37°C. The polymerization or clotting can possibly be carried out at various temperatures. For example, in a first step the polymerization is carried out at a first temperature, while in a second step, the polymerization is carried out at a temperature higher than the first temperature. For example, in the first step, the temperature of the reaction medium is lower than 30°C, preferably lower than 25°C, for example comprised between 15 and 25°C, while in the second step, the temperature of the reaction medium is higher than 30°C, for example comprised between 35 and 50°C, most preferably 37°C or about 37°C. Possibly, the temperature of the reaction medium is increased during the reaction, for example according to a substantially continuous path. For example, the reaction starts at a temperature lower than 20°C or equal to 20°C, and is progressively carried out at a temperature higher than 20°C, for example at an end temperature of about 37°C.

The fibrin-comprising material has, advantageously before its treatment like e.g. compression and/or stretching, an osmolarity greater than 50 mosm, most specifically greater than 100 mosm, preferably greater than 200 mosm, most preferably greater than 250 mosm, for example comprised between 250 and 400 mosm. The fibrin containing clot before its compression or stretching has advantageously an optical density lower than 1 Absorbance Unit Full Scale (=AUFS), advantageously lower than 0.5 AUFS.

The osmolarity has been measured by using the apparatus FISKE 2400 OSMOMETER (Fiske Associates) according to the following method: A sample is supercooled several degrees below the freezing point. The heat of fusion liberated allows sample temperature to rise a temporary "liquid-solid" equilibrium. Said equilibrium is by definition the freezing point of the solution, which is depending on the salt content, said salt content being determined by osmolarity measurements. The osmolarity is equal to the osmoles of solute per Kg of pure solvent. The optical density has been measured at a wavelength of 800 nm (Absorbance Unit Full Scale).

When using calcium complexing agent for the preparation of the biopolymer comprising material, the polymerization, advantageously with an at least partial cross-linking, is carried out in presence of an amount of calcium complexing or blocking agent, preferably an anticoagulant, sufficient for having a clotting time of more than 30 seconds, advantageously of more than 60 seconds, most preferably of more than 200 seconds, said clotting time being measured in the apparatus "BFT II" of DADE BEHRING (Germany) at 37°C. This apparatus operates according to the opto-mechanical measuring principle (measure of a turbidity). A light beam passes through a plastic cuvette containing 0.5 ml of the solution to be analyzed, onto a photodetector. The change of intensity of the transmitted light is converted into an electric signal. A stir bar is placed in the cuvette or cup so as to ensure homogeneity of the solution placed in the cuvette or cup.

The amount of calcium complexing or blocking agent can be determined by the man skilled in the art by successive tests for adding different amounts of calcium or blocking agent.

When the structure of the invention, after its stretching followed possibly by a drying step, is lyophilized, the lyophilization step is advantageously carried out at a temperature of less than 40°C and at a pressure of less than 0.4 10⁵ Pa. For said lyophilization, it can be worthwhile to add some specific additive(s), such as glycerol, fatty acid, etc. Said additive(s) is preferably added when preparing the fibrin-comprising material.

The lyophilization is advantageously carried out at different temperatures below -10°C. For example, the lyophilization is first carried out at a temperature below than -40°C, and then at a temperature comprised between -40°C and -10°C. According to a possible embodiment, the lyophilization is carried out at a temperature varying substantially continuously from a temperature below -40°C up to a temperature comprised between -40°C and -10°C.
For example, the lyophilization is carried out in several steps, such as lowering the temperature to about -58°C and maintaining said temperature during a period of time (for example from 1 to 30 hours, advantageously from 1 to 15 hours) while creating a vacuum, then increasing the temperature from -58°C to -20°C or -30°C while maintaining the vacuum, then by maintaining the temperature of - 20°C or - 30°C while maintaining the vacuum (for example from 5 to 100 hours), then increasing the temperature to more than 20°C, while maintaining the vacuum.

According to an advantageous embodiment, the fibrin-containing clot is prepared from material having a low albumin content, for example the said biopolymer solution contains less than 5 % by weight of albumin, with respect to the weight of the fibrin-fibrinogen material.

In the process of the invention, the fibrin-comprising material can be polymerized with a partial cross-linking. Partial cross-linking means for example a cross-linking of less than 50% by weight of the fibrin-comprising material, advantageously of 0.5 to 40%. In a preferred embodiment the above-mentioned degree of cross-linking is related to the fibrin content of the material.

When using fibrin or fibrinogen material, the fact that the fibrin or fibrinogen material is polymerized with a partial cross-linking with the factor XIII can be observed by an electrophoresis SDS - polyacrylamide gel by the absence of the γ-band detectable in the fibrin - fibrinogen starting material and by the intensity of the γ-γ band of the material after polymerization. The electrophoresis of reduced protein using 4-15% gradient polyacrylamide gels showed that for fibrin with complete cross-linking, there is no residual monomeric γ chains remaining and the cross linked fibrin showed characteristic β and γγ polymer chains as prominent (substantially no α chain is remaining).

The intensity of the γ-γ band is correlated to the cross-linking rate. It means that, when the intensity of the γ-γ band is low, the cross-linking rate of the fibrin or fibrinogen is low. In order to determine the cross-linking rate in the process of the invention, solutions with different fibrinogen content were cross-linked not in presence of calcium inhibiting agent and the intensity of the γ-γ band of the cross-linked fibrinogen solutions is determined by SDS analysis for having a reference. The intensity of the γ-γ band of the fibrinogen polymerized in presence of the calcium complexing agent is then compared with the reference, so as to determine the amount of cross-linked fibrinogen, and therefore the % of cross-linked fibrinogen and the % of polymerized (not cross-linked) fibrinogen. The amount of cross-linked fibrinogen in the structure of the invention is considered as being the amount of fibrinogen cross-linked not in presence of a calcium blocking agent (in a reference solution) having substantially the same intensity for the γ-γ band.

The invention relates also to a fibrin-comprising tube, thread or an article of any other shape which has not been stretched, but which is suitable for stretching. Said shape like e.g. a tube, thread or article is prepared by compressing a solution comprising fibrinogen material and thrombin, preferably in a mould additionally provided with a central mandrel. Preferably, the solution comprising fibrinogen material and thrombin is compressed in the mould while the polymerization is carried out.

According to a specific process, a photo-activatable thrombin is used, so as to ensure that the polymerization and cross-linking occurs substantially only in the mould for obtaining the shaped product.

According to a first embodiment, particles containing activatable thrombin and fibrinogen material or a mixture of particles containing respectively activatable thrombin and fibrinogen material or a mixture thereof are put in a mould (preferably after a homogeneous mixing of the particles). The particles placed in the mould are wetted or moistened and the thrombin is activated for initiating the polymerization. Possibly, the particles are moistened or wetted before to be cast or placed in the mould. After polymerizing and preferably removal of the unpolymerized fibrinogen, the shaped product can be stretched.

According to a second embodiment, layers of particles containing activatable thrombin and fibrinogen material or of a mixture of particles containing respectively activatable thrombin and fibrinogen material or a mixture thereof are superimposed in a mould (preferably after a homogeneous mixing of the particles). After each placement of a layer, the layer is moistened or wetted and the thrombin is activated at the place where fibrin structure is required. Possibly, so as to avoid the wetting step, a suspension of particles or a paste of particles is used, instead of a mixture of dry particles. After polymerization of all the superimposed layer, the polymerized product is removed and washed so as to remove the unpolymerized fibrinogen and inactive thrombin. The polymerized product is then possibly stretched.

When using dry or substantially dry particles in the latter embodiment, it is also possible to control the wetting step, so as to moisten or wet substantially only the area or zone where fibrin is required.

According to still a further possible process using activatable thrombin, a fibrin-comprising material, e.g. a clot, is prepared and compressed. Then, possibly during the compression step, specific activating (for example light) ray(s) is (are) directed towards specific area or zone of the compressed material so as to initiate the polymerization of fibrinogen at specific areas or zones of the material. After polymerization and possibly removal of the unpolymerized fibrinogen material, the fibrin-comprising product can then be stretched.

According to a detail of the process, the process further comprises a drying step.

The invention relates also to a process for the manufacture of a shaped article made at least partly of fibrin material, in which an aqueous solution containing fibrinogen material and thrombin in an inactivated form is provided, the amount of water present in the solution being advantageously such that after activating the thrombin and polymerization of the fibrinogen-containing material into a gel, substantially no water can be removed when submitting the gel to a centrifugation of 1,000 rounds per minute (advantageously 2,000 rounds per minute, preferably more than 2,500 rounds per minute), and in which the thrombin present in the solution is at least partly activated. The thrombin present in the solution is advantageously at least partly activated when submitting the solution to e.g. compression, advantageously in a mould or in dies.

The fibrin-comprising, shaped article can thereafter be submitted to a stretching and/or drying process, the stretching being preferably carried out during a drying step. The fibrin shaped article can also be first at least partly dried, and then stretched.

The fibrinogen-thrombin solution can be prepared by mixing water with particles selected from the group consisting of mixture of fibrinogen containing particles and inactive thrombin containing particles, particles containing fibrinogen material and inactive thrombin, and mixtures thereof.

The invention further relates to a process for making a shaped article made at least partly from fibrin or comprising at least partly a fibrin according to the invention. The article can be made from a mixture of particles containing at least particles selected from the group consisting of mixture of fibrinogen containing particles and inactive thrombin containing particles, particles containing fibrinogen material and inactive thrombin, and mixtures thereof. The mixture can be compressed or compacted, advantageously in a mould or in dies, in which the compressed or compacted particles are wetted or moistened, and in which the thrombin is at least partly activated. The fibrin-comprising, shaped article can thereafter be submitted to a stretching and/or drying, the stretching being preferably carried out during a drying step. The fibrin-comprising, shaped article can also be first at least partly dried and then stretched.

According to another embodiment, the article can be made out of particles, wherein the wetted or moistened particles are cast in a mould or extruded through dies, in which for at least part of the cast or extruded particles, thrombin is at least partly activated.
The fibrin shaped article can thereafter be submitted to a stretching and/or drying, the stretching can be carried out during a drying step. Possibly the fibrin shaped article is first at least partly dried, and then stretched. Advantageously, the mixture of particles is at least partly compressed or compacted in the mould or dies, and in which thrombin is at least partly activated for the compressed or compacted mixture.

The process can include the formation of a plurality of layers.

Another process of the invention is a process for making a shaped article made at least partly of fibrin, in which a fibrinogen-inactive thrombin solution or gel or wetted cake is prepared, and in which thrombin of the solution or gel is locally activated so as to form fibrin bonds at the place where thrombin is activated. Possibly after a washing step, the fibrin shaped article can thereafter be submitted to a stretching and/or drying, the stretching being possibly carried out during a drying step. Possibly the fibrin shaped article is first at least partly dried, and then stretched.

The compressed tube or thread or article is advantageously at least partly cross-linked in a solution containing a cross-linking agent, said cross-linking step being carried out before the drying step. The tube or thread or article is for example made of fibrin and of a compound selected from the group consisting of fibrinogen, chondroitin-4 sulfate, dermatan sulfate, keratan sulfate, hyaluronic acid, chitosan, chitin, alginate, laminin, elastin, fibronectin, collagen, organic polymer, peptide, derivatives thereof and mixtures thereof.

According to an advantageous embodiment, the tube or thread or article is prepared from a fibrinogen or fibrin solution containing advantageously less than 10 IU/ml thrombin, advantageously less than 5 IU/ml thrombin, preferably less than 2 IU/ml thrombin, most preferably less than 1 IU/ml.

According to a possible embodiment, the tube or thread or article is prepared from a fibrin solution containing at least 3 mg/ml, advantageously at least 5 mg/ml, preferably at least 10 mg/ml fibrinogen material. The fibrin or fibrinogen solution contains advantageously a calcium inhibiting agent, preferably in a concentration sufficient for having a clotting time of more than 30 seconds, for example of more than 60 seconds, most preferably of more than 120 seconds.

According to a possible embodiment, the tube or thread or article is prepared from a fibrin or fibrinogen solution containing at least a compound selected from the group consisting of fibrin, fibrinogen material, chondroitin-4 sulfate, dermatan sulfate, keratan sulfate, hyaluronic acid, chitosan, chitin, alginate, laminin, elastin, fibronectin, collagen, and mixtures thereof, and at least an additive.

According to a preferred embodiment, the tube or thread or article is prepared from a fibrinogen or fibrin solution containing photo-activatable thrombin, the thrombin being most preferably activated when the fibrin-fibrinogen solution is in the mould. The tube or thread or article prepared by said process can thereafter be coated and/or submitted to a stretching.

### DESCRIPTION OF EXAMPLES

### Example 1

To an aqueous solution containing 100 mg fibrinogen/ml and having a pH of 7.1, an albumin content of less than 5% by weight of the weight of fibrinogen, a factor XIII content of about 0.1 IU per mg fibrinogen, 1 ml of a phosphate buffer solution (PBS) per ml of the aqueous fibrinogen-containing solution (composition of the PBS buffer solution: NaCl 8g/l; KCl 200mg/l; Na₂HPO₄1.15g/l; KH₂PO₄ 200mg/l) was added. The pH of the so prepared buffered fibrinogen-containing solution was 7.2, while the fibrinogen content was 50 mg/ml. A solution containing 1 lU thrombin per ml (the thrombin solution was reconstituted from thrombin powder and PBS) was added to said buffered solution at the rate of 1 ml thrombin solution per ml of fibrinogen solution. When using this amount of PBS, the clotting time of the reaction mixture was 430 seconds at 37°C, said clotting time being measured by using the apparatus "Dade Behring BFT II" ® (version 1.2 - date of issue of the technical book October 1999) of Dade Behring, Germany. In said apparatus, a plastic vial containing 0.5 ml of the solution was placed in the apparatus for ensuring an agitation (by means of the stir bar) and a substantially constant temperature of the sample to be tested. The apparatus enables the detection of any small whirls or clots present in the sample due to the stirring bar.

The solution containing fibrinogen material and thrombin had an osmolarity of about 450 mosm, said osmolarity being measured by using the apparatus FISKE 2400 OSMOMETER (Fiske Associates) according to the following method:

A sample is supercooled several degrees below the freezing point. The heat of fusion liberated allows sample temperature to rise a temporary "liquid-solid" equilibrium. Said equilibrium is by definition the freezing point of the solution. Which is depending on the salt content, said salt content being determined by osmolarity measurements. The osmolarity is equal to the osmoles of solute per Kg of pure solvent.

The polymerization and partial cross-linking of the so formed fibrin were carried out in a tube having an inner diameter of about 2 mm, so as to obtain a tube containing a hydrogel, said hydrogel having an optical density (measured at a wavelength of 800 nm) of 0.255 AUFS (absorbance unit full scale).

The tube containing the hydrogel was compressed with a pressure of 2 kg/m².

During said compression, water is expelled from the hydrogel and a fibrin-containing thread is formed.

Thereafter the shaped thread is removed from the tube and stretched in air up to a diameter of 200 µm.

As the PBS buffer contains phosphate, the structure was enriched in P. The weight ratio P/fibrinogen material of the structure is about 0.3%.

The structure was washed, under aseptic conditions, with sterile and pyrogen-free water and can possibly further be sterilized by methods known per se, for example by γ-radiation (35 KGray).

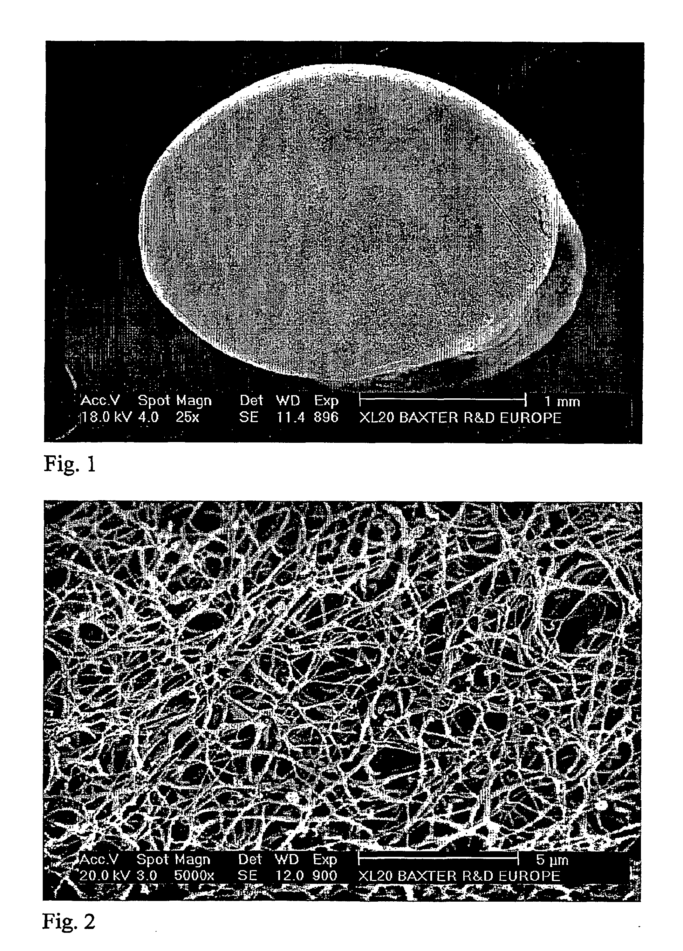

Figure 1 is a cross-section view (SEM, scanning electron microscope) of the fibrin-containing thread removed from the tube, before its stretching. Said thread has a diameter of about 2 mm.

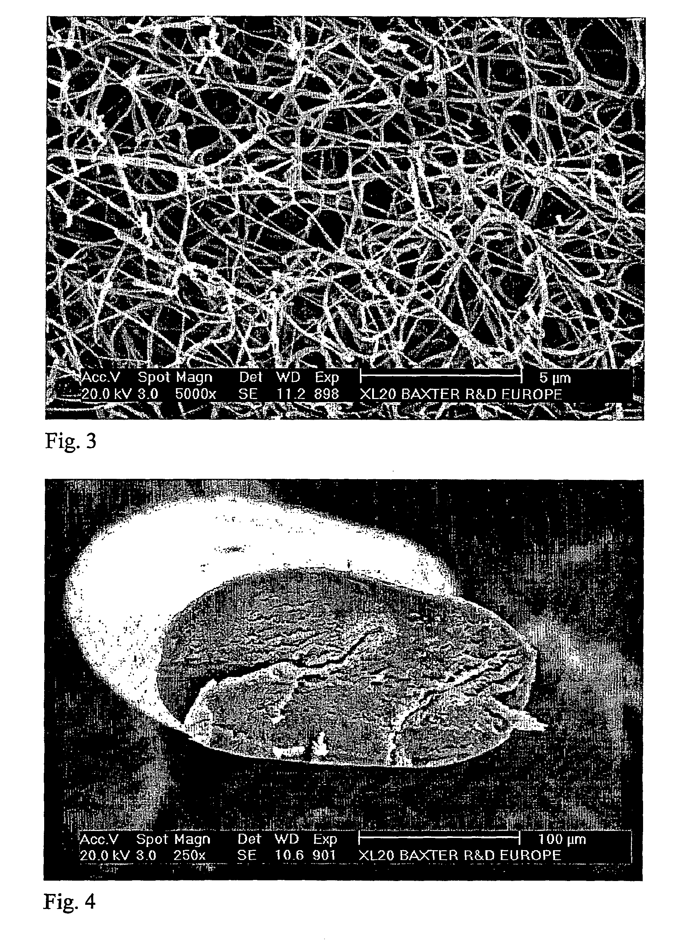

Figure 2 is an enlarged view (SEM, scanning electron microscope) of the outer wall of the thread of Figure 1, while Figure 3 is a cross-section view (SEM, scanning electron microscope) of the thread of Figure 1. From said figures 2 and 3, it appears that the fibrin density is greater in the outer wall than in the cross-section and that the pore size is greater in the cross-section than in the outer wall.

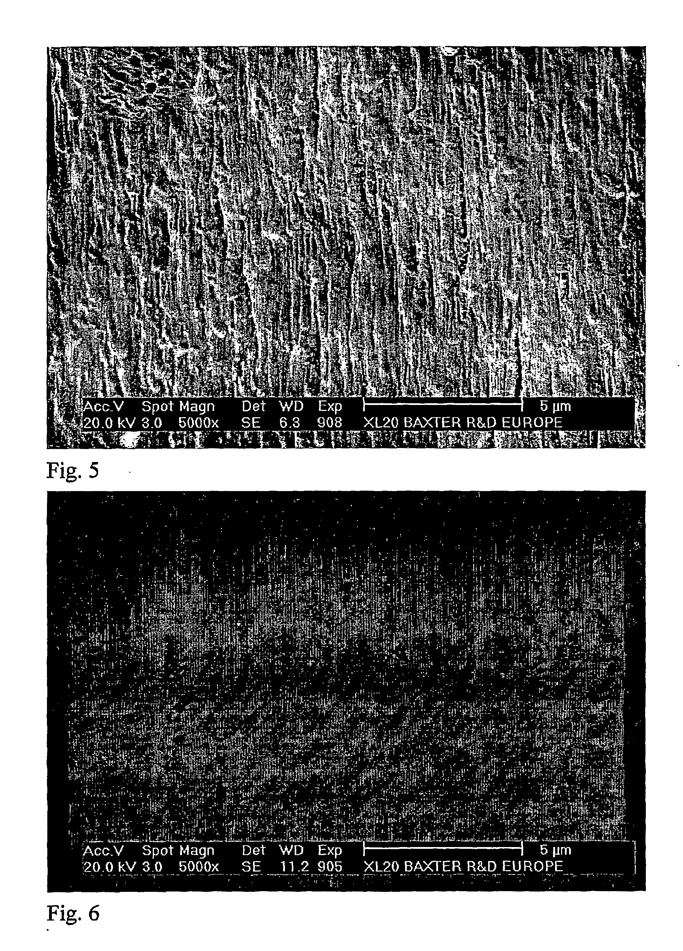

After stretching the thread in air (stretching direction : longitudinal axis of the thread), the diameter of the thread was reduced to about 200 µm (Figure 4). Figure 5 is an enlarged view (SEM, scanning electron microscopy) of the outer surface of the stretched thread of Figure 4, while Figure 6 is an enlarged view (SEM, scanning electron microscopy) of the cross-section of said stretched thread. The fibrin material was aligned with respect to the stretching direction (longitudinal axis of the thread), whereby substantially no fibrin could be seen in the cross-section view, while most of the fibrin was visible on the outer wall. After stretching, the pore size of the thread was reduced to less than 0.1 µm in the cross-section view.

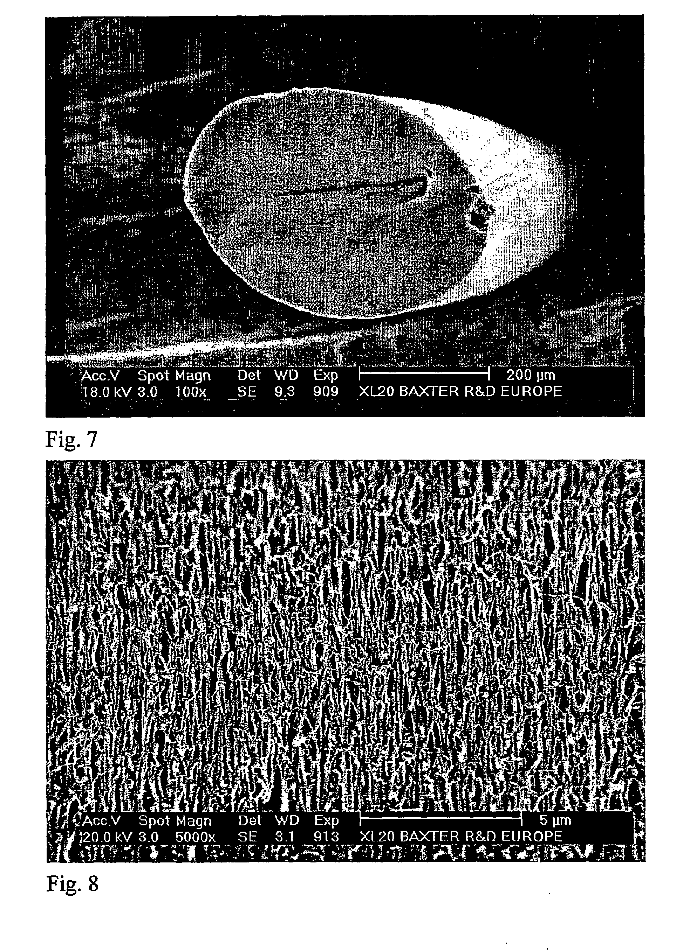

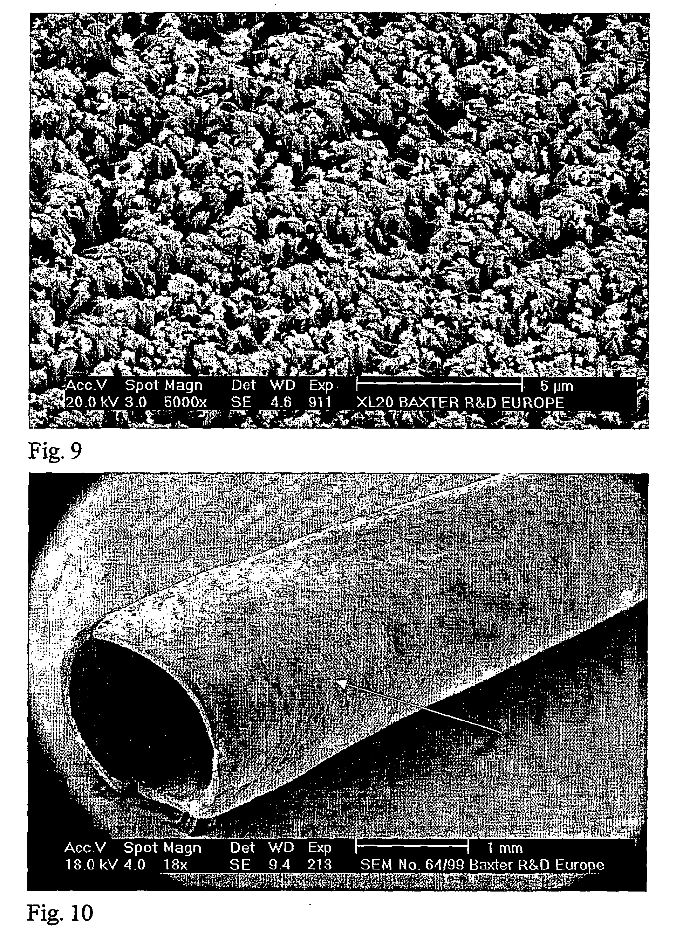

A part of the thread of Figure 1 was stretched in a fixative solution (glutaraldehyde: 2% by weight). After said stretching, the thread had a diameter of about 400 µm. Figure 7 is a cross-section view of the thread after stretching in the fixative solution. Figure 8 is an enlarged view of the outer surface of the thread, while Figure 9 is an enlarged view of the cross-section of the thread of Figure 7. These figures show that fibers are aligned parallel to the stretching direction (longitudinal axis of the thread) and that the void volume between the fibers is lower than 0.2 µm.

It was observed that the dried stretched fibrin-containing thread recovered their mechanical properties after hydration.

### Example 2

Example 1 is repeated, except that the solution fibrinogen - thrombin was injected in a mould with a cylindrical chamber (having a diameter of about 4 mm) provided with a central mandrel (having a diameter of about 2 mm). The solution was polymerized in said mould. After polymerization, the mandrel provided with a layer of polymerized fibrin was removed from the mould and submitted to a compression (pressure: 2 kg/cm²). Said compression was carried out by placing the mandrel provided with the layer in a pressurized chamber. During said compression step some water was expelled out of the layer.

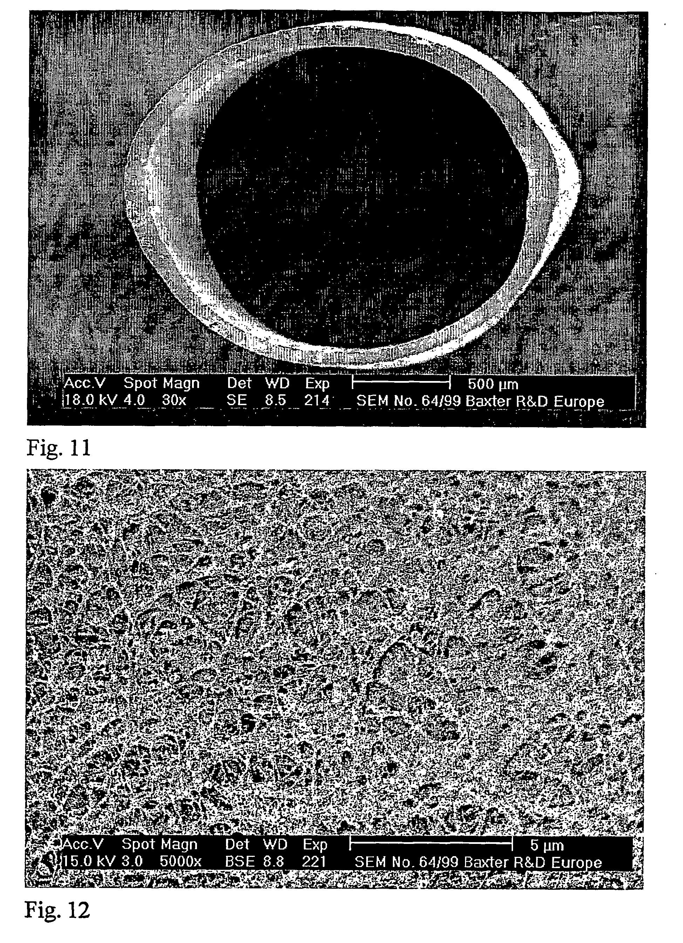

Figure 10 is a perspective view of the tube, while figure 11 is a cross-section view of the tube. The diameter of the tube was about 2 mm, while the thickness of the wall was about 100 µm.

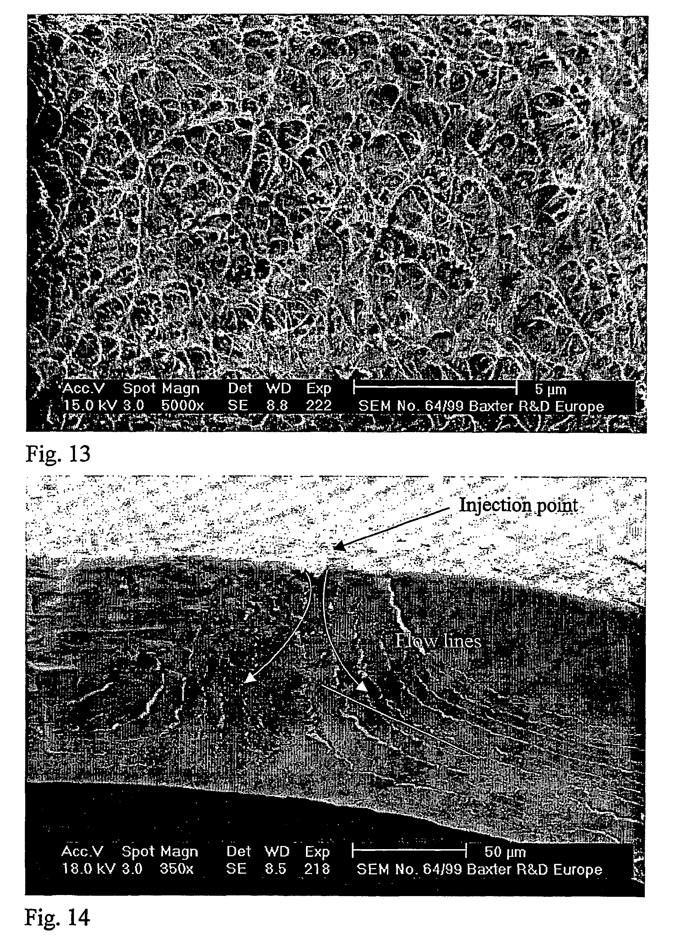

Figure 12 shows the fibrin structure along the inner side of the tube, while Figure 13 shows the fibrin structure along the outer side of the tube. Figure 14 is a cross-section view of the tube at the injection point of the fibrinogen-thrombin solution. Said figure shows the flow lines of the fibrin material in the mould.

Thereafter, the fibrin tube was submitted to a stretching.

After said stretching, the tube had an inner diameter of about 1 mm and a wall thickness of about 50 µm.

### Examples 3 to 14

Example 1 was repeated, except that the fibrinogen-containing solution was mixed with a solution containing another biomaterial or other biomaterials. Said biomaterial solution was added to the fibrinogen solution, before adding the thrombin solution. The biomaterial solution to be added to the fibrinogen solution had a biomaterial content of 25 mg/ml and was added to the fibrinogen solution (containing 25 mg fibrin/ml) at the rate 1:1 (1 ml biomaterial solution added per 1 ml fibrinogen solution).

The following table gives the biomaterial added.

| **Example** | **Biomaterial added** |
|---|---|
| 3 | chondroitin-4 sulfate |
| 4 | dermatan sulfate |
| 5 | keratan sulfate |
| 6 | hyaluronic acid |
| 7 | chitosan |
| 8 | alginate |
| 9 | laminin |
| 10 | fibronectin |
| 11 | elastin |
| 12 | collagen |
| 13 | collagen 50%+ fibronectin 50% |
| 14 | fibronectin 50% + alginate 50% |

### Examples 15 to 25

Example 1 has been repeated, except that additives have been added to the solution before its polymerization. Said additives were added to the solution of fibrinogen before its mixing with the thrombin solution.

The following table gives the additives and the quantity of additives (mg/ml of fibrinogen solution) added.

| **Example** | **Additive** | **Amount mg/ml of solution** |
|---|---|---|
| 15 | bone growth factor BMP | 1 |
| 16 | osteoblast | 1 |
| 17 | indomethacin (anti-inflammatory) | 3 |
| 18 | paracetamol (pain killer) | 3 |
| 19 | glycerol | 5 |
| 20 | a frazzled polypeptide (compound preventing a graft rejection) | |
| 21 | calcium glycerophosphate | 2 |
| 22 | morphine | 1 |
| 23 | sulfamide | 2 |
| 24 | sodium polyethylene | 3 |
| 25 | Zocor® | 2 |

### Example 26

Example 1 was repeated, except that the stretching of the fibrin-containing thread was carried out in an aqueous bath containing glutaraldehyde (concentration: 2% by weight).

### Example 27

Example 2 was repeated, except that the stretching of the fibrin-containing thread was carried out in an aqueous bath containing glutaraldehyde (concentration: 2% by weight).

### Example 28

Example 1 was repeated, except that the solution was polymerized on a support plate so as to produce a fibrin-containing layer having a thickness of about 2 mm.

The layer was stretched in a nitrogen atmosphere in one direction (direction parallel to the plane defined by the layer) so as to reduce the thickness of the film to 250 µm.

### Example 29

Example 28 was repeated, except that the layer was stretched in a first direction up to lowering the thickness to 1 mm, and then in a second direction perpendicular to the first direction, said first and second directions being parallel to the plane defined by the layer. After said second stretching, the film had a thickness of about 500 µm. The film produced in this method is not an embodiment of the invention.

### Example 30

Example 28 was repeated, except that the layer was locally embossed, whereby the film was provided with several stretched portions, distant the one from the other. The stretched portions had a thickness of about 1 mm.

### Examples 31 to 33

Examples 28 to 30 were repeated, except that the stretching was carried out in a bath containing glutaraldehyde (concentration: 2% by weight) and that the stretched film was dried in a nitrogen atmosphere.

### Example 34

A polymerized thread (not stretched) was prepared as in example 1. The thread was then used as the central mandrel of a mould such as in example 2.

A collagen solution (as disclosed in US 4,963,146, for example in column 12) was then injected in the mould.

After polymerization the two-layered thread having an outer diameter of 3 mm was stretched so as to reduce the diameter of the thread to about 500 µm.

### Examples 35 and 36

The stretched thread of example 1, the stretched tube of example 2 and the stretched film of example 28 were coated with a collagen layer having a thickness of about 250 µm.

### Example 37

The threads of example 1, after drying, were used for knitting a fabric.

The fabric was then dipped in a glutaraldehyde bath (2% by weight) so as to cross-link the threads together at their crossing points.

The fabric was then washed, dried and sterilized by γ radiation at low temperature (-25°C).

The sterile and pyrogen-free fabric can be used as a support for proteins for removing a substance from the blood of a human subject. The coupling of the protein(s) on the support can be performed in accordance to the method disclosed in US 5,817,528, the content of which is incorporated by reference.

The protein coupled to the structure is for example the protein A.

Other proteins which could be coupled to the structure are: Streptococcus Protein G, Anti-human immunoglobulin antibodies, anti-LDL antibodies, etc.

The structure provided with the said protein(s) can be used in a column for removing immunoglobulin G or M or A or E, LDL or lipoprotein A.

### Example 38

The stretched thread of example 1 after drying and sterilization was filled with a drug or an active agent.

For said filling, the structure was immersed in a solution containing the drug or active agent in a dissolved form. Thereafter the solvent is removed for example by lyophilization. The preferred solvent is water or an aqueous medium (aqueous medium containing a polyethylene glycol, for example a PEG with a low molecular weight, PEG 400, PEG 600, etc.).

Example of possible active agents or drugs are: antibody, antimicrobial agent, agent for improving the biocompatibility of the structure, proteins, anticoagulants, anti-inflammatory compounds, compounds reducing graft rejection, living cells, cell growth inhibitors, agents stimulating endothelial cells, antibiotics, antiseptics, analgesics, antineoplastics, polypeptides, protease inhibitors, vitamins, cytokine, cytotoxins, minerals, proteins, interferons, hormones, polysaccharides, genetic materials, proteins promoting or stimulating the growth and/or attachment of endothelial cells on the cross-linked fibrin, growth factors, cell growth factors, growth factors for heparin bond, tannic acid, nerve growth factor, neurotrophic factor (NTFs), neurothrophin 3 (NT3), brain derived NTF (BDNTF), cilary NTF (CNTF), substances against cholesterol, pain killers, collagen, osteoblasts, chondroblasts, chondrocytes, osteoclasts, hematpoeitic cells, stromal cells, osteoprogenitor cells, drugs, anti coagulants, poly DL lactate, alginate, recombinant material, triglycerides, fatty acids, C₁₂-C₂₄ fatty acids, drugs, substances against cholesterol, pain killers, etc. and mixtures thereof.

The filled threads (acting as a drug delivery system) can possibly thereafter be cut into granules.

### Example 39

Example 1 has been repeated for the preparation of a hydrogel. However, in example 39, half of the volume of PBS used in example 1 has been replaced by a PAS (=Platelet Additive Solution) buffer solution.

Due to the use of a mixture of PAS and PBS buffers, the volume of the porous layer 2 after its hydration was substantially equal to the volume of the layer before its hydration.

### Examples 40 to 43

Example 1 has been repeated except that the compression was controlled so as to control the water volume removed from the hydrogel.

The hydrogel formed in the examples 40, 41, 42 and 43 had a water loss of respectively 5% by volume, 10% by volume, 15% by volume and 20% by volume. A water loss of 5% by volume means that a volume of water corresponding to 5% of the volume of the initial hydrogel (before stretching) passed through the filter and was therefore removed from the hydrogel.

### Example 44

Example 1 has been repeated except that citric acid was added to the solution containing the fibrin-fibrinogen-thrombin so that the polymerization is carried out at a pH of 4.5.

After polymerization and stretching, the thread was washed for removing the excess of citric acid.

### Examples 45 to 48

Example 1 has been repeated, except that the concentrations of fibrinogen material and of the thrombin of the solution fibrinogen - thrombin varied.

The following table gives the concentration of fibrinogen and thrombin, after mixing the fibrinogen solution with the thrombin solution.

| **Example** | **Fibrinogen (mg/ml)** | **Thrombin IU/ml** |
|---|---|---|
| 45 | 50 | 1 |
| 46 | 100 | 1 |
| 47 | 200 | 1 |
| 48 | 100 | 2 |

### Example 49

Example 1 has been repeated, except that, after the compression step, the shaped thread was sterilized by a gamma radiation at a temperature of -25°C. Thereafter, the sterilized shaped thread was stretched in air up to a diameter of 200 µm.

### Example 50

Example 1 was repeated, except that a photoactivatable thrombin (Cryolife) was used.

The fibrinogen - photoactivatable thrombin was compressed in the cylindrical mould, the wall of which was transparent. The mixing and compression were carried out in a dark room or through a light absorbing device. When compressed, the thrombin was activated by light.

The compressed tube was thereafter removed from the mould and submitted to a stretching so as to form a thread with a diameter of 200 µm.

### Example 51

Dry particles of fibrinogen material and photactivatable thrombin have been mixed together. The mixed particles had a ratio fibrinogen/photoactivatable thrombin of about 100 mg/20lU.

The mixture was mixed with water, the amount of which was such that after activating the thrombin and polymerization (with cross-linking) of the fibrinogen material, the fibrin product has substantially no water removal when submitting it to a centrifugation of 2,000 rounds per minute.

The aqueous mixture was cast in a mould and compressed. During said compression step, thrombin was activated.

The shaped product was then subjected to a stretching.

### Example 52

Dry particles of fibrinogen material and photactivatable thrombin have been mixed together. The mixed particles had a ratio fibrinogen/photoactivatable thrombin of about 100 mg/20IU.

The mixture of particles was cast in a mould. Thereafter, the particles present in the mould were wetted with an amount water, such that after activating the thrombin and polymerization (with cross-linking) of the fibrinogen material, the fibrin product has substantially no water removal when submitting it to a centrifugation of 2,000 rounds per minute.

The wetted mixture was compressed. During said compression step, thrombin was activated.

The shaped product was then subjected to a stretching and then to a drying.

### Example 53

Dry particles of fibrinogen material and photactivatable thrombin have been mixed together. The mixed particles had a ratio fibrinogen/photoactivatable thrombin of about 100 mg/20/U.

A layer of particles was placed on a supporting face. The layer is wetted and the layer is submitted to local activation of the thrombin at the place where the fibrin product has to be shaped.

Thereafter a new layer of particles is placed on the preceding layer that forms a supporting face. Said new layer is then wetted and submitted to local activation of the thrombin where the fibrin product has to be shaped.

The process is then continued layer per layer.

The shaped product was then subjected to a washing, then to a stretching, and finally to a drying.

### Example 54

Dry particles of fibrinogen material and photactivatable thrombin have been mixed together. The mixed particles had a ratio fibrinogen/photoactivatable thrombin of about 100 mg/20lU.

The mixture of particles was cast and compressed in a mould. Thereafter, the particles present in the mould were wetted with an amount of water, such that after activating the thrombin and polymerization (with cross-linking) of the fibrinogen material, the fibrin product has substantially no water removal when submitting it to a centrifugation of 2,000 rounds per minute.

Thrombin was then activated for initiating the polymerization.

The shaped product was then subjected to a stretching and then to a drying.

In the processes of the invention, especially in the examples, in which a compression step is exerted on the fibrinogen - thrombin solution or on the fibrin clot, the compression can be operated by placing the solution or clot under vacuum. This is advantageous for preventing the formation of voids or of large voids. Preferably, the vacuum is exerted during the polymerization, most preferably before and during the polymerization.

## Claims

1. An elongated biopolymer structure containing fibrin having at least a portion stretched in only one stretching direction wherein the elongated structure has a shape of a thread or tube.

2. The structure of claim 1 in which the structure further contains a material selected from the group consisting of fibrinogen, chondroitin-4 sulfate, dermatan sulfate, keratan sulfate, hyaluronic acid, chitosan, chitin, alginate, laminin, elastin, fibronectin, collagen, organic polymer, peptide, derivatives thereof, and mixtures thereof.

3. The structure of claim 1 in which the stretched portion of the structure is porous.

4. The structure of claim 1 in which the material of the stretched portion of the structure has at least two densities which are different from each other.

5. The structure of claim 4 in which the first density is at least 1.5 times the second density.

6. The structure of claim 4 in which the first density is at least 5 times the second density.

7. The structure of claim 1 in which the stretched portion has an outer diameter of less than 10 mm.

8. The structure of claim 1 in which the stretched portion has a shape of a tube with a central channel substantially parallel to the stretching direction, said central channel having a cross-section perpendicular to the stretched direction with a diameter of less than 15 mm.

9. The structure of claim 8 in which said tube has a wall thickness between 0.1 mm and 5 mm.

10. The structure of claim 1 in which the amount of fibrin in the resultant product is more than 50% of the components of the starting material.

11. The structure of claim 1 in which the elongated structure contains at least partly cross-linked fibrin.

12. Process for the preparation of an elongated biopolymer structure of claim 1, comprising the steps of;
providing a first component of a fibrinogen-containing material;
providing a second component of a substance having a capability to convert fibrinogen into fibrin;
optionally providing a structure of a biopolymer having a stretched portion in only one stretching direction in the first or second component; and
forming a fibrin-containing material by mixing the first component with the second component to form an elongated biopolymer structure containing fibrin having at least a portion stretched in only one stretching direction.

13. Process according to claim 12 wherein the biopolymer is selected from fibrin, fibrinogen, chondroitin-4 sulfate, dermatan sulfate, keratan sulfate, hyaluronic acid, chitosan, chitin, alginate, laminin, elastin, fibronectin, collagen, organic polymer, peptide, derivatives thereof, and mixtures thereof.

14. Process according to claim 12 wherein the stretching is sufficient to elongate the length of the fibrin-comprising material by at least 5%.

15. Process according to claim 12 which further comprises a drying step.

16. Process according to claim 12 wherein at least part of the fibrin-comprising material is stretched by mechanical or physical treatment.

17. Process according to claim 16 wherein the mechanical treatment is one of a compression or an extrusion and the physical treatment is one of an energy treatment or freeze-drying.

18. Process according to claim 12 in which the fibrin-comprising material is prepared in a mould or in dies, said material being thereafter stretched by a mechanical or physical treatment in said mould or dies.

19. Process according to claim 12 in which the fibrin-comprising material is at least partly stretched in a solution containing a cross-linking agent.

20. Process according to claim 12 in which the fibrin-comprising material is mechanically or physically treated in dies or in a mould so as to obtain an article having a shape of a thread or tube.

21. Process according to claim 12 in which the fibrin-comprising material contains free water, and in which at least part of said free water is removed before the mechanical or physical treatment is carried out.

22. Process according to claim 12 in which the fibrinogen-containing material contains at least one additional compound selected from the group consisting of fibrin, chondroitin-4 sulfate, dermatan sulfate, keratan sulfate, hyaluronic acid, chitosan, chitin, alginate, laminin, elastin, fibronectin, collagen, organic polymer, peptide, derivatives thereof, and mixtures thereof.

23. Process according to claim 12 in which the fibrin-containing material is prepared from a fibrinogen-containing material as the first component and a solution containing less than 10 IU/ml thrombin as the second component.

24. Process according to claim 12 in which the fibrin-containing material is prepared from a fibrinogen-containing material as the first component and a solution containing less than 1 IU/ml thrombin as the second component.

25. Process according to claim 12 in which the fibrin-containing material is prepared from a solution having a fibrinogen content of at least 3 mg/ml.

26. Process according to claim 12 in which the fibrin-containing material is prepared from a fibrinogen-containing solution containing a calcium complexing agent.

27. Process according to claim 12 in which the material from which the structure is made further contains at least an additive selected from the group consisting of protein, genetic material, anticoagulant, inorganic compound, growth factor, cells, anti-inflammatory compound, compound reducing graft rejection, cell growth inhibitor, antibiotic, antiseptic, analgesic, antineoplastic, chemotherapeutic, polypeptide, protease inhibitor, vitamin, cytokine, cytotoxin, interferon, hormone, antibody, antimicrobial agent, agent for improving the biocompatibility, derivatives thereof, and mixtures thereof.

28. Process according to claim 12 in which the fibrin-containing material is submitted to lyophilization, preferably after stretching.

29. Article comprising an elongated biopolymer structure of claim 1.

30. Thread or tube, obtainable by a process according to claim 12.

31. Thread or tube, of claim 30, which is rolled around an axis perpendicular to a stretching direction.

32. A process for the manufacture of a shaped article comprising an elongated biopolymer structure according to claim 12 using an aqueous fibrinogen-containing solution as the first component and thrombin in an inactive form as the second component wherein the amount of water present in the solution is such that after activation of the thrombin and polymerization of the fibrinogen-containing material into a gel, no water can be removed when submitting the gel to a centrifugation of 1,000 rounds per minute.

33. The process of claim 32 in which the thrombin present in the solution is at least partly activated when submitting the solution to a mechanical or physical treatment, advantageously in a mould or in dies.

34. A process for making a shaped article comprising an elongated biopolymer structure made according to claim 12, said process further comprising mixing substances containing particles selected from the group consisting of fibrinogen, inactive thrombin, derivatives thereof and mixtures thereof, subjecting the mixture to a mechanical or physical treatment, advantageously in a mould or in dies, wetting said particles, activating said thrombin, and obtaining said shaping article.

## Patentansprüche

1. Langgestreckte Biopolymerstruktur, enthaltend Fibrin mit mindestens einem gestreckten Teil in nur eine Streckrichtung, wobei die langgestreckte Struktur die Form eines Fadens oder einer Röhre aufweist.

2. Struktur nach Anspruch 1, in welcher die Struktur weiter ein Material, ausgewählt aus der Gruppe, bestehend aus Fibrinogen, Chondroitin-4-Sulfat, Dermatansulfat, Keratanzulfat, Hyaluronsäure, Chitosan, Chitin, Alginat, Laminin, Elastin, Fibronectin, Collagen, organischem Polymer, Peptid, Derivaten davon und Gemischen davon, enthält.

3. Struktur nach Anspruch 1, in welcher der gestreckte Teil der Struktur porös ist.

4. Struktur nach Anspruch 1, in welcher das Material des gestreckten Teils der Struktur mindestens zwei Dichten aufweist, welche unterschiedlich voneinander sind.

5. Struktur nach Anspruch 4, in welcher die erste Dichte mindestens das 1,5-fache der zweiten Dichte beträgt.

6. Struktur nach Anspruch 4, in welcher die erste Dichte mindestens das 5-fache der zweiten Dichte beträgt.

7. Struktur nach Anspruch 1, in welcher der gestreckte Teil einen äußeren Durchmesser von weniger als 10 mm aufweist.

8. Struktur nach Anspruch 1, in weicher der gestreckte Teil eine Form einer Röhre mit einem zu der Streckrichtung im wesentlichen parallelen zentralen Kanal aufweist, wobei der zentrale Kanal einen zu der Streckrichtung senkrechten Querschnitt mit einem Durchmesser von weniger als 15 mm aufweist.

9. Struktur nach Anspruch 8. in welcher die Röhre eine Wanddicke zwischen 0,1 mm und 5 mm aufweist.

10. Struktur nach Anspruch 1, in welcher der Antell an Fibrin im resultierenden Produkt mehr als 50% der Komponenten des Ausgangsmaterials beträgt.

11. Struktur nach Anspruch 1, in welcher die langgestreckte Struktur mindestens teilweise quervernetztes Fibrin enthält

12. Verfahren zur Herstellung einer langgestreckten Biopolymerstruktur nach Anspruch 1, umfassend die Schritte:
des Bereitstellens einer ersten Komponente eines Fibrinogen-enthaltenden Materials,
des Bereitstellens einer zweiten Komponente einer Substanz mit einer Befähigung, Fibrinogen in Fibrin umzuwandeln,
gegebenenfalls des Bereitstellens einer Struktur eines Biopolymers mit einem gestreckten Teil in nur einer Streckrichtung in der ersten oder zweiten Komponente, und
des Bildens eines Fibrin-enthaltenden Materials durch Mischen der ersten Komponente mit der zweiten Komponente unter Bildung einer langgestreckten, Fibrin-enthltenden Biopolymerstruktur, welche mindestens einen in nur einer Streckrichtung gestreckten Teil aufweist.

13. Verfahren nach Anspruch 12, wobei das Biopolymer aus Fibrin, Fibrinogen, Chondroitin-4-Sulfat, Dermatansulfat, Keratansulfat, Hyaluronsäure, Chitosan, Chitin, Alginat, Laminin, Elastin, Fibronectin, Collagen, organischem Polymer, Peptid, Derivaten davon und Gemischen davon, ausgewählt ist.

14. Verfahren nach Anspruch 12, wobei das Strecken ausreicht, um die Länge des Fibrin-umfassenden Materials um mindestens 5% langzustrecken.

15. Verfahren nach Anspruch 12, welches weiter einen Trocknungsschritt umfasst.

16. Verfahren nach Anspruch 12, wobei mindestens ein Teil des Fibrin-umfassenden Materials durch mechanische oder physikalische Behandlung gestreckt wird.

17. Verfahren nach Anspruch 16, wobei das mechanische Behandeln eines einer Kompression oder einer Extrusion ist und das physikalische Behandeln eines einer Energiebehandlung oder Gefriertrocknen ist.

18. Verfahren nach Anspruch 12, in welchem das Fibrin-umfassende Material in einer Form oder in Düsen hergestellt wird, wobei das Material danach durch eine mechanische oder physikalische Behandlung in der Form oder den Düsen gestreckt wird.

19. Verfahren nach Anspruch 12, in welchem das Fibrin-umfassende Material mindestens teilweise in einer ein Vernetzungsmittel enthaltenden Lösung gestreckt wird.

20. Verfahren nach Anspruch 12, in welchem das Fibrin-umfassende Material mechanisch oder physikalisch in Düsen oder in einer Form behandelt wird, sodass ein Gegenstand mit einer Form eines Fadens oder einer Röhre erhalten wird.

21. Verfahren nach Anspruch 12, in welchem das Fibrin-umfassende Material freies Wasser enthält, und in welchem mindestens ein Teil des freien Wassers entfernt wird, bevor die mechanische oder physikalische Behandlung durchgeführt wird.

22. Verfahren nach Anspruch 12. in welchem das Fibrinogen-enthaltende. Material mindestens eine zusätzliche Verbindung, ausgewählt aus der Gruppe, bestehend aus Fibrin, Chondroitin-4-Sulfat, Dermatansulfat, Keratansulfat, Hyaluronsäure, Chitosan, Chitin, Alginat, Laminin, Elastin, Fibronectin, Collagen, organisches Polymer, Peptid, Derivaten davon und Gemischen davon, enthält,

23. Verfahren nach Anspruch 12, in welchem das Fibrin-enthaltende Material aus einem Fibrinogen-enthaltenden Material als der ersten Komponente und einer Lösung, enthaltend weniger als 10 IU/ml Thrombin, als der zweiten Komponente hergestellt wird.

24. Verfahren nach Anspruch 12, in welchem das Fibrin-enthaltende Material aus Fibrinogen-enthaltendern Material als der ersten Komponente und einer Lösung, enthaltend weniger als 1 IU/ml Thrombin, als der zweiten Komponente hergestellt wird.

25. Verfahren nach Anspruch 12, in welchem das Fibrin-enthaltende Material aus einer Lösung mit einem Fibrinogen-Gehalt von mindestens 3 mg/ml hergestellt wird.

26. Verfahren nach Anspruch 12, in welchem das Fibrin-enthaltende Material aus einer Fibrinogen-enthaltenden Lösung hergestellt wird, welche ein Calcium-komplexierendes Mittel enthalt.

27. Verfahren nach Anspruch 12, in welchem das Material, aus welchem die Struktur hergestellt wird, weiter mindestens ein Additiv, ausgewählt aus der Gruppe, bestehend aus Protein, genetischem Material, Antikoagulans, anorganischer Verbindung, Wachstumsfaktor, Zellen, entzündungshemmender Verbindung, Verbindung zur Verringerung der Transplantatabstossung, Zellwachstums-Hemmstoff, Antibiotikum, Antiseptikum, Analgetikum, Antineoplastikum, Chemotherapeutikum, Polypeptid, Protease-Hemmstoff, Vitamin, Cytokin, Cytotoxin, Interferon, Hormon, Antikörper, antimikrobiellern Mittel, Mittel zur Erhöhung der Biokompatibilität, Derivaten davon und Gemischen davon, enthält.

28. Verfahren nach Anspruch 12, in welchem das Fibrin-enthaltende Material einer Lyophilisation unterzogen wird, vorzugsweise nach dem Strecken.

29. Gegenstand, enthaltend eine langgestreckte Biopolymerstruktur nach Anspruch 1.

30. Faden oder Röhre, erhältlich.durch ein Verfahren nach Anspruch 12.

31. Faden oder Röhre nach Anspruch 30, welche(r) um eine zu einer Streckrichtung senkrechte Achse gewickelt ist.

32. Verfahren zur Herstellung eines geformten Gegenstands, umfassend eine langgestreckte Biopolymerstruktur nach Anspruch 12. unter Verwendung einer wässrigen Fibrinogen-enthaltenden Lösung als die erste Komponente und Thrombin in einer inaktiven Form als die zweite Komponente, wobei die Menge von In der Lösung vorliegendem Wasser derart ist, dass nach Aktivierung des Thrombins und Polymerisation des Fibrinogen-enthaltenden Materials zu einem Gel kein Wasser entfernt werden kann, wenn das Gel einer Zentrifugation mit 1000 Umdrehungen pro Minute ausgesetzt ist.

33. Verfahren nach Anspruch 32, in welchem das in der Lösung vorliegende Thrombin mindestens teilweise aktiviert wird, wenn die Lösung einer mechanischen oder physikalischen Behandlung, vorzugsweise in einer Form oder in Düsen, ausgesetzt wird.

34. Verfahren zur Herstellung eines Formgegenstands, umfassend eine langgestreckte Biopolymerstruktur nach Anspruch 12, wobei das Verfahren weiter das Mischen von Substanzen, welche Teilchen, ausgewählt aus der Gruppe, bestehend aus Fibrinogen, inaktivem Thrombin, Derivaten davon und Gemischen davon, enthalten.
das Unterziehen des Gemisches einer mechanischen oder physikalischen Behandlung, vorteilhafterweise in einer Form oder in Düsen,
das Benetzen der Teilchen,
das Aktivieren des Thrombins und
das Erhalten des Formgegenstands umfasst.

## Revendications

1. Structure biopolymère allongée renfermant de la fibrine ayant au moins une partie s'étendant dans une seule direction d'étirement dans laquelle la structure allongée a la forme d'un fil ou d'un tube.

2. Structure selon la revendication 1 dans laquelle la structure contient en outre une substance sélectionnée parmi le groupe constitué par le fibrinogène, la chondroïtine-4 sulfate, le dermatan sulfate, le kératan sulfate, l'acide hyaluronique, le chitosane, la chitine, l'alginate, la laminine, l'élastine, la fibronectine, le collagène, un polymère organique, un peptide, des dérivés de ceux-ci, et des mélanges de ceux-ci.

3. Structure selon la revendication 1 dans laquelle la partie étirée de la structure est poreuse.

4. Structure selon la revendication 1 dans laquelle la substance de la partie étirée de la structure a au moins deux densités qui sont différentes l'une de l'autre.

5. Structure selon la revendication 4 dans laquelle la première densité est égale à au moins 1,5 fois la seconde densité.

6. Structure selon la revendication 4 dans laquelle la première densité est égale à au moins 5 fois la seconde densité.

7. Structure selon la revendication 1 dans laquelle la partie étirée a un diamètre extérieur de moins de 10 mm.

8. Structure selon la revendication 1 dans laquelle la partie étirée a la forme d'un tube avec un canal central substantiellement parallèle à la direction d'étirement, ledit canal central ayant une section perpendiculaire à la direction d'étirement avec un diamètre de moins de 15 mm.

9. Structure selon la revendication 8 dans laquelle ledit tube a une épaisseur de paroi entre 0,1 mm et 5 mm.

10. Structure selon la revendication 1 dans laquelle la quantité de fibrine dans le produit résultant est supérieure à 50 % des composants de la substance de départ.

11. Structure selon la revendication 1 dans laquelle la structure allongée contient au moins de la fibrine réticulée partiellement.

12. Processus pour la préparation d'une structure biopolymère allongée selon la revendication. 1, comprenant les étapes de :
fournir un premier composant d'une substance renfermant un fibrinogène ;
fournir un second composant d'une substance ayant une capacité à convertir le fibrinogène en fibrine ;
de manière optionnelle fournir une structure d'un biopolymère ayant une partie étirée dans une seule direction d'étirement dans le premier ou le second composant ; et
former une substance renfermant de la fibrine en mélangeant le premier composant avec le second composant afin de former une structure biopolymère allongée renfermant de la fibrine ayant au moins une partie étirée dans une seule direction d'étirement.

13. Processus selon la revendication 12 dans lequel le biopolymère est sélectionné parmi la fibrine, le fibrinogène, la chondroïtine-4 sulfate, le dermatan sulfate, le kératan sulfate, l'acide hyaluronique, le chitosane, la chitine, l'alginate, la laminine, l'élastine, la fibronectine, le collagène, un polymère organique, un peptide, des dérivés de ceux-ci, et des mélanges de ceux-ci.

14. Processus selon la revendication 12 dans lequel l'étirement est suffisant pour allonger la longueur de la substance renfermant la fibrine d'au moins 5 %.

15. Processus selon la revendication 12 qui comprend en outre une étape de séchage.

16. Processus selon la revendication 12 dans lequel au moins une partie de la substance renfermant la fibrine est étirée au moyen d'un traitement mécanique ou physique.

17. Processus selon la revendication 16 dans lequel le traitement mécanique est soit une compression soit une extrusion et le traitement physique est soit un traitement énergétique soit une lyophilisation.

18. Processus selon la revendication 12 dans lequel la substance renfermant la fibrine est préparée dans un moule ou dans des matrices, ladite substance étant ci-après étirée par un traitement mécanique ou physique dans ledit moule ou lesdites matrices.

19. Processus selon la revendication 12 dans lequel la substance renfermant la fibrine est au moins en partie étirée dans une solution contenant un agent de réticulation.

20. Processus selon la revendication 12 dans lequel la substance renfermant la fibrine est traitée de manière mécanique ou physique dans des matrices ou dans un moule de manière à obtenir un article ayant une forme de fil ou de tube.

21. Processus selon la revendication 12 dans lequel la substance renfermant la fibrine contient de l'eau libre, et dans lequel au moins une partie de ladite eau libre est retirée avant que le traitement mécanique ou physique soit réalisé.

22. Processus selon la revendication 12 dans lequel la substance renfermant la fibrine contient au moins un composé additionnel sélectionné parmi le groupe constitué par la fibrine, la chondroïtine-4 sulfate, le dermatan sulfate, le kératan sulfate, l'acide hyaluronique, le chitosane, la chitine, l'alginate, la laminine, l'élastine, la fibronectine, le collagène, un polymère organique, un peptide, des dérivés de ceux-ci, et des mélanges de ceux-ci.

23. Processus selon la revendication 12 dans lequel la substance renfermant la fibrine est préparée à partir d'une substance renfermant du fibrinogène comme premier composant et une solution contenant moins de 10 IU/ml de thrombine comme second composant.

24. Processus selon la revendication 12 dans lequel la substance renfermant la fibrine est préparée à partir d'une substance renfermant du fibrinogène comme premier composant et une solution contenant moins de 1 IU/ml de thrombine comme second composant.

25. Processus selon la revendication 12 dans lequel la substance renfermant la fibrine est préparée à partir d'une solution ayant une teneur en fibrinogène d'au moins 3 mg/ml.

26. Processus selon la revendication 12 dans lequel la substance renfermant la fibrine est préparée à partir d'une solution renfermant du fibrinogène contenant un agent complexant du calcium.

27. Processus selon la revendication 12 dans lequel la substance à partir de laquelle la structure est faite contient en outre au moins un additif sélectionné parmi le groupe constitué par une protéine, du matériel génétique, un anticoagulant, un composé inorganique, un facteur de croissance, des cellules, un composé anti-inflammatoire, un composé réduisant les rejets de greffes, un inhibiteur de croissance de cellules, un antibiotique, un antiseptique, un analgésique, un anticancéreux, un agent chimiothérapeutique, un polypeptide, un inhibiteur de protéase, une vitamine, une cytokine, une cytotoxine, un interféron, une hormone, un anticorps, un agent antimicrobien, un agent pour l'amélioration de la biocompatibilité, des dérivés de ceux-ci, et des mélanges de ceux-ci.

28. Processus selon la revendication 12 dans lequel la substance renfermant la fibrine est soumise à la lyophilisation, de préférence après l'étirement.

29. Article comprenant une structure biopolymère allongée selon la revendication 1.

30. Fil ou tube, pouvant être obtenu par un processus selon la revendication 12.

31. Fil ou tube, selon la revendication 30, qui est enroulé autour d'un axe perpendiculaire à une direction d'étirement.

32. Processus pour la fabrication d'un article profilé comprenant une structure biopolymère allongée selon la revendication 12 utilisant une solution aqueuse renfermant un fibrinogène comme premier composant et de la thrombine sous une forme inactive comme second composant dans lequel la quantité d'eau présente dans la solution est telle qu'après l'activation de la thrombine et la polymérisation de la substance renfermant le fibrinogène en un gel, de l'eau ne peut pas être retirée lorsque le gel est soumis une centrifugation à 1 000 tours par minute.

33. Processus selon la revendication 32 dans lequel la thrombine présente dans la solution est au moins en partie activée lorsque la solution est soumise à un traitement mécanique ou physique, de manière avantageuse dans un moule ou dans des matrices.

34. Processus pour fabriquer un article profilé comprenant une structure biopolymère allongée selon la revendication 12, lequel processus comprenant en outre de mélanger les substances contenant des particules sélectionnées parmi le groupe constitué par le fibrinogène, la thrombine inactive, les dérivés de ceux-ci et les mélanges de ceux-ci, de soumettre le mélange à un traitement mécanique ou physique, de manière avantageuse dans un moule ou dans des matrices, de mouiller lesdites particules, d'activer ladite thrombine, et d'obtenir ladite particule de profilage.
